# EUROPEAN PATENT SPECIFICATION

(11) **EP 3 254 721 B1**
(45) Date of publication and mention of the grant of the patent: **19.08.2020**
(21) Application number: 16746140.9
(22) Date of filing: 04.02.2016
(51) Int. Cl.: A61M 16/06, A61M 16/08, A61M 16/20

(54) **FACE MASK**
GESICHTSMASKE
MASQUE FACIAL

(30) Priority: 05.02.2015 CN 201510064930
(43) Date of publication of application: 13.12.2017
(73) Proprietor: Resgood Medical Co., Ltd, Shenzhen, Guangdong 518117 (CN)
(72) Inventor: LEI, Mingchu, Shenzhen, Guangdong 518117 (CN); FAN, Zhongcheng, Shenzhen, Guangdong 518117 (CN); FEI, Shichao, Shenzhen, Guangdong 518117 (CN)
(74) Representative: Petraz, Gilberto Luigi
(86) International application number: PCT/CN2016/073476
(87) International publication number: WO 2016/124145

(56) References cited:
- WO-A1-00/38772
- WO-A1-2013/170290
- WO-A1-2014/053987
- CN-A- 102 014 999
- CN-A- 104 208 789
- CN-A- 104 225 750
- CN-A- 104 587 580
- CN-A- 104 623 784
- CN-U- 202 314 828
- CN-U- 203 577 098
- CN-U- 204 655 725
- CN-U- 204 655 726
- US-A1- 2006 130 844
- US-A1- 2014 238 400
- US-B2- 8 596 275

## Description

### FIELD OF THE INVENTION

The present disclosure relates to a mask, and more particularly to a medical respiratory mask used in corporation with an air delivery system.

### BACKGROUND OF THE INVENTION

In the treatment directed to sleep respiratory disorders such as obstructive sleep apnea, it is usual to adopt a positive airway pressure therapy, i.e., delivery of a positive pressure air to the respiratory airway of patient through a respiratory device. Such respiratory device generally comprise a positive airway pressure device, i.e., a Continuous Positive Airway Pressure (CPAP) device or a bilevel positive airway pressure (Bi-PAP) device, an air delivery pipe and a respiratory interface of a patient, wherein the respiratory interface of a patient is generally a full mask or an oral-nasal mask. A mask generally comprises a connector connected to an air delivery pipe, an elbow, an anti-asphyxia valve, a shell, a cushion, a headgear and a frame. The connector, the anti-asphyxia valve, the elbow, the shell and the cushion are connected successively, the frame is secured on the shell, and both ends of the headgear are latched to the frame. During application, the mask is secured by the headgear onto the mouth & nose area of a patient, the cushion is in contact with the skin in a peripheral profile area of the mouth & nose of the patient, the cushion is adhered to the skin in the contact area to form air tightness by the action of the headgear and the pressure difference between inside and outside of the mask so that the positive pressure air is delivered to the respiratory airway of the patient. During application, the comfort level, the stability and the air tightness when a mask is in contact with the skin are three important indicators and will directly affect user experience and therapeutic effects of a patient. A common breathing mask is described for example in the document CN-A-104208789. Document US-B-8596275 describes a known a patient interface with an adjustable cushion. A known nasal mask system is also described in the document WO-A-2013/170290. Document WO-A-00/38772 discloses a known anti-asphyxia valve.

A common mask is in contact with the oral or nasal part of a person, and long-time wearing will result in a decrease in comfortability. Moreover, the cushions of the existing masks cannot satisfactorily match the nasal profiles of most patients. For example, one type mask cannot satisfy the application requirements of both the patients with a low nose bridge and the patients with a high nose bridge at different age groups, thus leading to poor universality.

### SUMMARY OF THE INVENTION

Accordingly, it is necessary to provide a mask with better universality and ability to improve wearing comfortability

The invention is defined in the appended independent claim 1. Preferred embodiments are defined in the dependent claims.

A mask comprising:
a connector;
an anti-asphyxia valve connected to the connector;
an elbow connected to the anti-asphyxia valve;
a shell connected to the elbow;
a cushion disposed at an end of the shell away from the elbow, wherein the cushion defines a first opening and a second opening, the first opening faces towards the shell, the second opening is away from the shell, and an edge region adjacent to the second opening of the cushion defines a flexible pad; the cushion comprises a nasal contact area and an oral contact area, wherein the nasal contact area forms concavely a buffer structure which is an elongated structure extending from a top end of the nasal contact area towards the oral contact area along both sides, the buffer structure comprises a first buffer member, a second buffer member and an inwardly concave surface connected to the first buffer member and the second buffer member, the first buffer member is adjacent to the first opening, the second buffer member is adjacent to the flexible pad, and the inwardly concave surface can deform to adjust the distance between the first buffer member and the second buffer member;
a frame latched to the shell, wherein the frame comprises an engaging portion, an arc-shaped bridge extending from the engaging portion towards an end, and a connecting end formed at the end of the arc-shaped bridge away from the engaging portion, the engaging portion defines an engaging hole and respectively extends outwardly from both sides of the engaging hole to form a pulling portion, the engaging hole being detachably sleeved on the shell; and
a headgear for connecting the connecting end and two pulling portions of the frame to assist in wearing of the mask,
wherein, when the mask is being worn, two pulling portions of the frame are suspendingly placed on a face part of a wearer and the connecting end is suspendingly placed on a forehead part so that the frame does not contact the skin of face or forehead of patients.

As the inwardly concave surface at the buffer structure can deform, the distance between the first buffer member and the second buffer member can be adjusted so as to enable the cushion to flexibly adapt to the nasal contact areas of wearers with different heights of nose bridges. Moreover, without affecting the air tightness between the cushion and the facial skin of a wearer, the pressure applied by the cushion onto the nose bridge part of a patient during wearing is significantly reduced, thus avoiding formation of a red imprint on the wearer's face after wearing for a long time and improving wearing universality and comfortability. Further, the connecting member of the frame is suspendingly placed on the face or forehead of a person, so that it is not in contact with a human body and avoids an uncomfortable feeling caused by long-time wearing.

### BRIEF DESCRIPTION OF THE DRAWINGS

The above and other objects, features and advantages of the present disclosure will become more apparent by describing in detail embodiments thereof with reference to the accompanying drawings. The components in the drawings are not necessarily drawn to scale, the emphasis instead being placed upon clearly illustrating the principles of the present disclosure. Moreover, in the drawings, like reference numerals designate corresponding parts throughout the views.
FIG. 1 is a schematic perspective view of a mask according to an embodiment;
FIG. 2 is a schematic exploded view of a mask shown in FIG. 1;
FIG. 3 is a schematic view of a combination of a connector, an anti-asphyxia valve and an elbow of the mask shown in FIG. 1;
FIG. 4 is a schematic exploded view of the connector, the anti-asphyxia valve and the elbow shown in FIG. 3;
FIG. 5 is a cross-sectional view of the connector, the anti-asphyxia valve and the elbow shown in FIG. 3;
FIG. 6 is a schematic perspective view of a shell of the mask shown in FIG. 1;
FIG. 7 is a schematic view of a combination of the shell and the cushion of the mask shown in FIG. 1;
FIG. 8 is a cross-sectional view of the shell and the cushion shown in FIG. 7;
FIG. 9 is a schematic perspective view of a cushion of the mask shown in FIG. 1;
FIG. 10 is a schematic perspective view of another perspective of the cushion shown in FIG. 9;
FIG. 11 is a cross-sectional view of the cushion shown in FIG. 9;
FIG. 12 is a schematic perspective view of a frame of the mask shown in FIG. 1;
FIG. 13 is a schematic perspective view of another perspective of the frame shown in FIG. 12;
FIG. 14 is a cross-sectional view of the frame shown in FIG. 12;
FIG. 15 is a schematic view of a combination of the shell, the cushion and the frame of the mask shown in FIG. 1;
FIG. 16 is a cross-sectional view of the frame and the shell taken along line XVI-XVI in FIG. 15;
FIG. 17 is a cross-sectional view of the frame and the shell taken along line XVII-XVII shown in FIG. 15;
FIG. 18 is a schematic perspective view of the mask shown in FIG. 1 being worn.

### DETAILED DESCRIPTION OF THE EMBODIMENTS

The present invention will be described in the following with reference to the accompanying drawings and the embodiments. Preferably, embodiments are presented in the drawings. However, numerous specific details are described hereinafter in order to facilitate a thorough understanding of the present disclosure.

It should be noted that when an element is called "secured" in another element, it can be directly on another element or there may also exist an intermediate element. When an element is considered as "connected" to another element, it can be directly connected to another element or there may exist an intermediate element as the same time. The terms "vertical", "horizontal", "upper", "lower", "left", "right" and similar expressions used in the text are only for the purpose of explaining.

Unless otherwise defined, all technical and scientific terms used herein have the same meaning as commonly understood by one of ordinary skill in the art to which this invention belongs. The terms used in the specification of the present invention in the text are only for the purpose of describing specific embodiments rather than limiting the present invention. The term "and/or" used in the text includes any and all combinations of one or more relevant items listed.

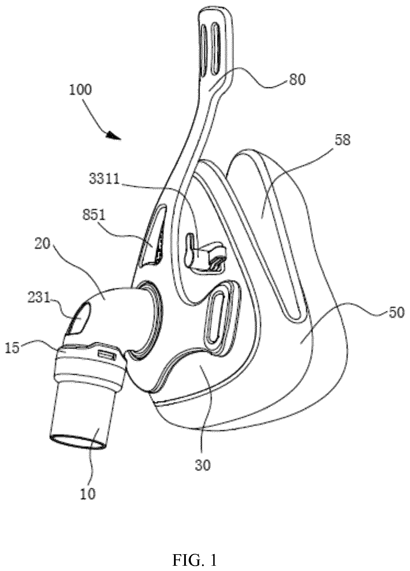

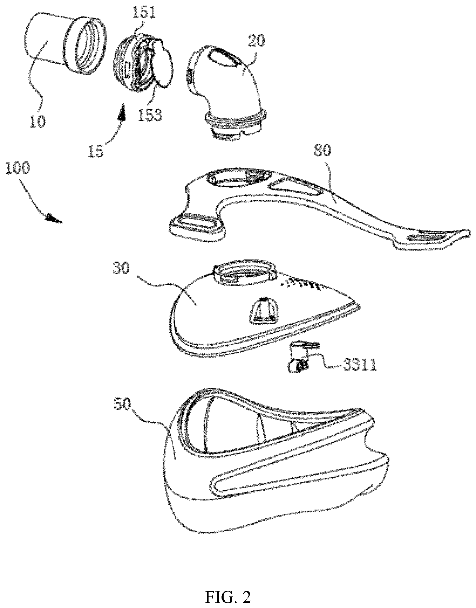

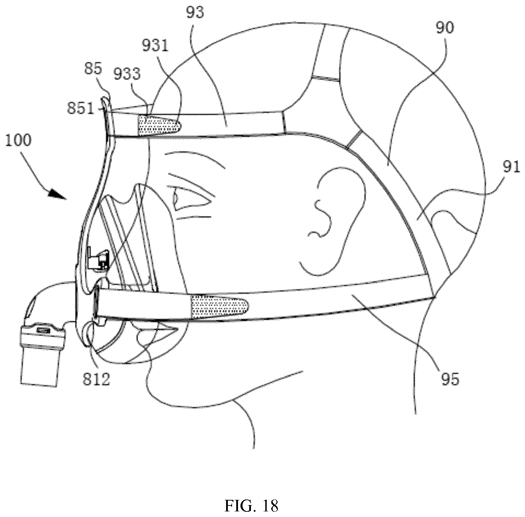

Referring to FIG. 1 and FIG. 2, a mask 100 in an embodiment is a medical mask used for positive airway pressure (PAP) therapy, which comprises a connector 10, an anti-asphyxia valve 15, an elbow 20, a shell 30 and a cushion 50, a frame 80 disposed on the shell 30 and a headgear 90 connected to the frame 80 that are connected successively (referring to FIG. 18). The connector 10 is used to be connected to an air delivery pipe (not shown). The anti-asphyxia valve 15 is rotatably connected to the connector 10, and the elbow 20 is latched to the anti-asphyxia valve 15. The shell 30 is detachably latched to the elbow 20, and the cushion 50 is molded on the shell 30 by over-molding and is used to contact the periphery area of an oral or nasal profile of a wearer. The frame 80 is sleeved on the shell 30, and both ends of the headgear 90 are used to be connected to both sides of the frame 80 so as to secure the mask 10 to cover the oral and nasal contact areas of the wearer.

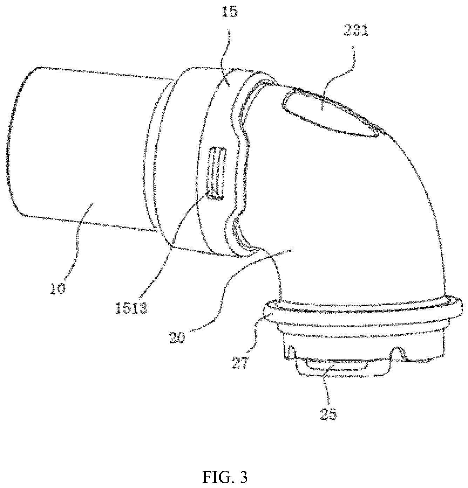

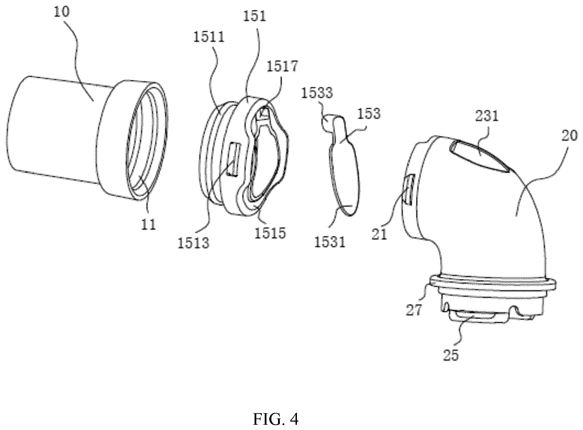

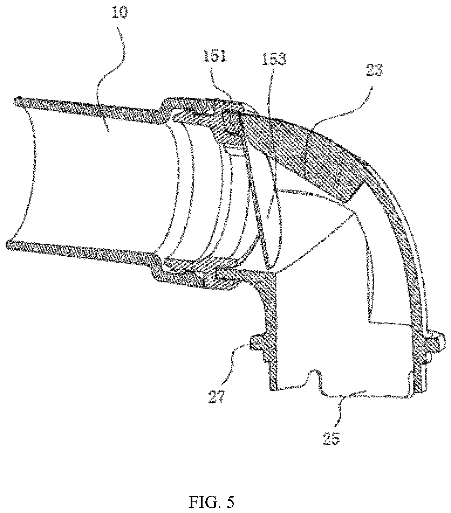

Also referring to FIG. 3 to FIG. 5, the connector 10 is a hollow tubular member, an end of which surrounds an inner periphery and forms inwardly an annular portion 11. The anti-asphyxia valve 15 comprises a valve body 151 and a valve membrane 153. The valve body 151 is annular, an end of which surrounds the periphery and protrudes outwardly to form a snap ring 1511, and the other end of which forms two latching holes 1513 at an outer side of the periphery and forms an annular supporting surface 1515 at an end. The valve body 151 further forms concavely a receiving hole 1517 at an inner side of an end where the supporting face 1515 resides. The snap ring 1511 is latched to the annular portion 11 of the connector 10 so that the valve body 151 is rotatably connected to the connector 10 and cannot be detached from the connector 10. The valve membrane 153 is made of a silicone rubber material, and comprises a sealing portion 1531 and a handle portion 1533 formed at the periphery of the sealing portion 1531. The sealing portion 1531 is a circular membrane with a uniform thickness in a range of 0.2mm to 0.8mm. The sealing portion 1531 is disposed at an end of the valve body 151 as a block. The handle portion 1533 has a greater thickness than that of the sealing portion 1531 and is secured by insertion into the receiving hole 1517 of the valve body 151.

The elbow 20 is in a bent shape, one end of which is inserted into the anti-asphyxia valve 15 and adhered to the supporting surface 1515. The elbow 20 has two fasteners 21 by protruding outwardly from the periphery. The two fasteners 21 are respectively inserted into two latching holes 1513 of the anti-asphyxia valve 15. The elbow 20 forms a joint face 23 which tilts with respect to an axis of the elbow 20 on an inner wall thereof adjacent to an end of the anti-asphyxia valve 15, and the elbow 20 further defines two anti-asphyxia holes 231 on the joint face 23, and the anti-asphyxia holes 231 penetrates a side wall of the elbow 20. The elbow 20 is symmetrically provided with a snap joint 25 disposed at an end thereof away from the connector 10, and a sealing ring 27 is formed around the periphery adjacent to the end.

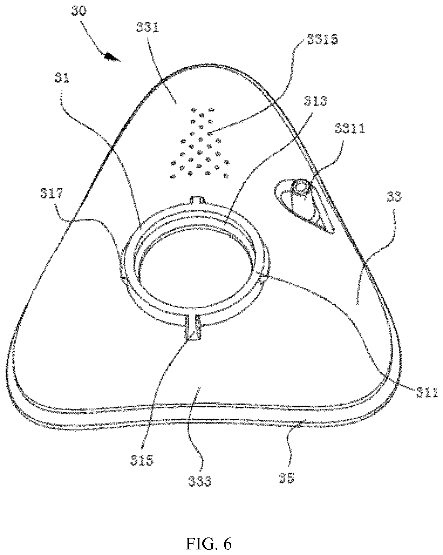

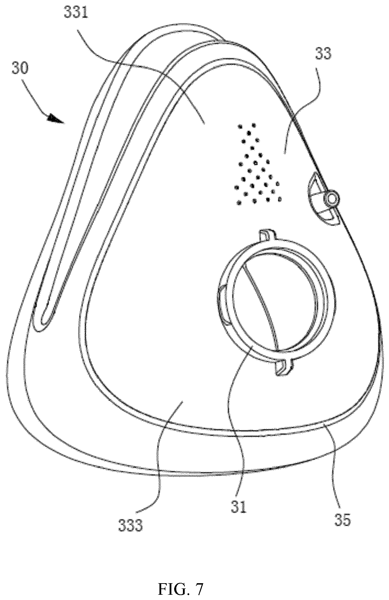

Also referring to FIG. 6 and FIG. 7, the front outline of the shell 30 is approximately shaped as an isosceles triangle. In the embodiment, the front outline of the shell 30 has a length of about 90mm to 100mm and a width of about 80mm to 85mm. The elbow 20 is latched to the shell 30 and is rotatable with respect to the shell 30. The shell 30 comprises a connector portion 31, a frame portion 33 and an interface portion 35 that are connected successively. The size of the shell 30 increases gradually along the connector portion 33 towards the interface portion 35. The connector portion 31 is annular, and rotatably sleeved on an end part of the elbow 20. An annular sealing surface 311 is formed at an end part of the connector portion 31 away from the shell portion 33, an annular snap rib 313 is formed by protruding inwardly an inner periphery of the connector portion 31, and two clamping positions 315 and two button positions 317 are formed by protruding outwardly from the outer periphery of the connector portion 31. The sealing surface 311 of the connector portion 31 is closely adhered to the sealing ring 27 of the elbow 20, and the snap rib 313 is latched to two snap joints 25 from one side of the sealing ring 27. The sealing ring 27 of the elbow 20 can ensure effective air tightness of the shell 30 with respect to the elbow 20 during rotation. Two clamping positions 315 of the shell 30 are symmetrically disposed, two button positions 317 are symmetrically disposed, and a connection line between the two clamping positions 315 is perpendicular to a connection line between the two button positions 317.

The cross-sectional profile of the shell portion 33 is a triangle, and the cross-sectional size of the shell portion 33 increases gradually from the connector portion 31 towards the interface portion 35. As illustrated in FIG. 6, the shell portion 33, according to an exterior profile, is divided into a first shell area 331 at an upper part and a second shell area 333 at a lower part, wherein the first shell area 331 forms an inner cavity that receives the nose of the wearer, and the second shell area 333 forms an inner cavity that receives the mouth area of the wearer. The first shell area 331 defines an oxygen pipe interface 3311 near the second shell area 333 to connect an oxygen pipe in particular conditions, e.g., in an oxygen-supply airway treatment. However, in normal conditions, the interface 3311 should be sealed with a sealing plug 3313 to avoid air leakage. The first shell area 331 defines a plurality of vent holes 3315 away from the second shell area 333. In this embodiment, the number of the vent holes 3315 is between 25 and 30. The vent holes 3315 are circular, and has an inner diameter of about 0.6mm to 0.9mm.

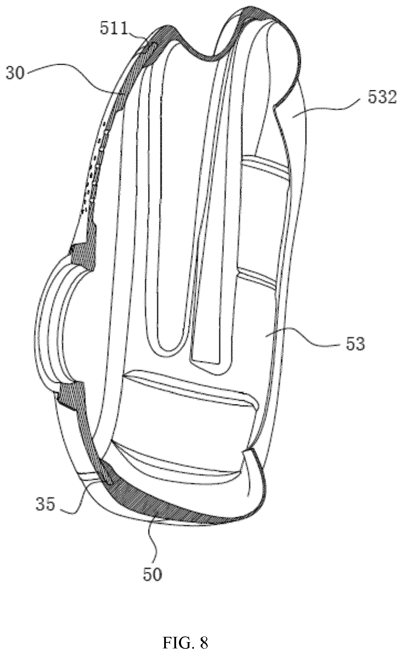

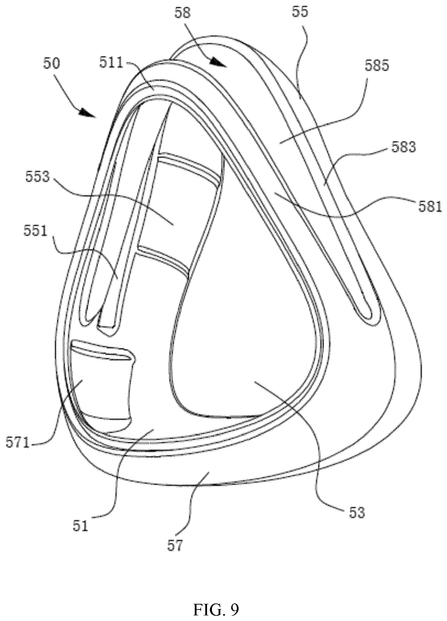

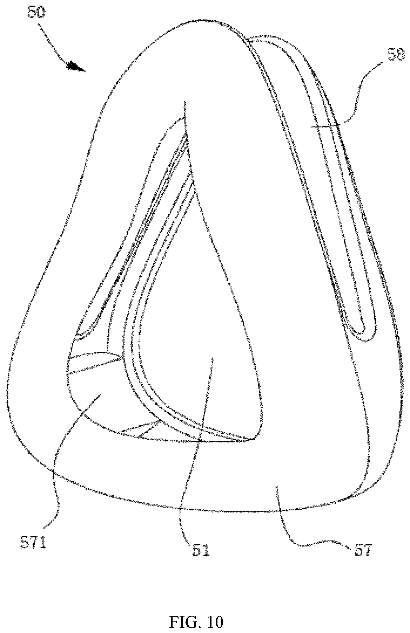

Referring to FIG. 8 to FIG. 11, the shape design of the cushion 50 conforms to ergonomics, and can match different oral and nasal profiles of a majority of patients. In this embodiment, the cushion 50 is made of a single-layered silicone rubber and is undetachably formed in the interface portion 35 of the shell 30. The silicone rubber material of the cushion 50 has a Shore hardness of about 40 degrees. The cushion 50 defines a first opening 51 and a second opening 53 at both ends thereof, respectively. The shape outlines of the first opening 51 and the second opening 53 are consistent with the front outlines of the mouth and nose of the wearer. The first opening 51 faces towards the shell 30, and the second opening 53 is away from the shell 30 and towards the mouth and nose of the wearer. the edge of the second opening 53 defines a flexible pad 532. In wearing, the flexible pad 532 will contact with the facial skin of the wearer along a profile of the nose bridge, the cheek and the underlip/chin of the wearer to form air tightness. The thickness of the cushion 50 is gradually reduced from the first opening 51 towards the second opening 53, with a smallest thickness of about 0.4mm. An embedded slot 511 is formed concavely on the periphery surrounding the first opening 51 of the cushion 50. The embedded slot 511 is provided on an end face of the cushion 51 towards the shell 30 and is closely sleeved on an interface portion 35 of the shell 30, i.e., on the periphery of the interface portion 35.

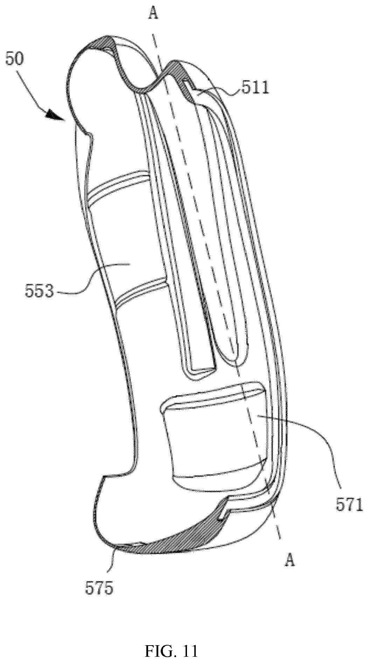

The cushion 50, according to the shape outline, is divided into a nasal contact area 55 in contact with the nose of the wearer and an oral contact area 57 in contact with the mouth. The nasal contact area 55 is at the same side as the first shell area 331 of the shell portion 33. The oral contact area 57 is at the same side as the second shell area 333 of the shell portion 33. The nasal contact area 55 concavely defines a buffer structure 58. The buffer structure 58 is an elongated structure extending from the top end of the nasal contact area 55 towards the oral contact area 57 along both sides. The buffer structure 58 comprises a first buffer member 581, a second buffer member 583 and an inwardly concave surface 585 connecting the first buffer member 581 and the second buffer member 583. The first buffer member 581 is close to the first opening 51, and the second buffer member 583 is close to the flexible pad 532. The first buffer member 581, the second buffer member 583 and the inwardly concave surface 585 are all symmetrically disposed with respect to an axis of the cross section of the cushion 50. An inwardly concave depth of the inwardly concave surface 585 gradually decreases from the middle part towards the first buffer member 581 and the second buffer member 583, and gradually decreases from the nasal contact area 55 towards the oral contact area 57. In this embodiment, the plane A (referring to FIG. 11) where the profile line of the inwardly concave surface 585 resides is inclined with respect to a central connection line between the first opening 51 and the second opening 53, and is gradually away from the second opening 53 towards the direction of the nasal contact area 55 along the oral contact area 57. The top end of the second buffer member 585 protrudes outwardly with respect to the first buffer member 581. The maximum distance between the first buffer member 581 and the second buffer member 585 is about 10mm to 16mm. The inwardly concave depth of the inwardly concave surface 585 is 10mm to 13mm, and the curvature radius of the cross-sectional profile thereof is 4mm to 40mm. The thickness throughout the inwardly concave surface 585 is uniform in the range of 0.4mm to 1.5mm. The distance between the first buffer member 581 and the second buffer member 583 of the buffer structure 58 can be freely adjusted within a certain range as the inwardly concave surface 585 is compressed and rebounded. When a patient with a higher nose bridge wears the mask 100, the distance between the first buffer member 581 and the second buffer member 583 of the buffer structure 58 on the cushion 50 will be reduced to automatically match the nose profile of the patient with the nose bridge, thereby improving adaptability thereof. Meanwhile, owing to the buffer function of the buffer structure 58, the pressure imposed by the cushion 50 on the nose bridge part of the patient can be significantly reduced when wearing, thereby avoiding formation of an red imprint on the wearer's face after wearing for a long time and improving wearing comfortability, without affecting the air tightness between the cushion 50 and the facial skin of the patient.

The nasal contact area 55 defines two thickening structural stiffeners 551 and two opening stiffeners 553 adjacent to the buffer structure 58. The two structural stiffeners 551 are symmetrically disposed in approximately middle part of the inner side of the nasal contact area 55. The opening stiffeners 553 are disposed symmetrically with one another, and located at the corresponding structural stiffeners 551 adjacent to the second opening 53. Two thickening first stiffeners 571 are formed at the inner side of the oral contact area 57 adjacent to the nasal contact area 55, and a second stiffener 575 is formed away from the nasal contact area 55 (referring to FIG. 11). Two first stiffeners 571 are symmetrically disposed and are respectively adjacent to the structural stiffeners 551 in the nasal contact area 55. The second stiffener 575 is located between two first stiffeners 571. In the present embodiment, the thickness of the structural stiffeners 551 is about 2.5mm to 3mm, and the thickness of the opening stiffeners 553 is about 1mm. The thickness of the first stiffeners 571 is about 2.5mm to 4mm, and the thickness of the second stiffener 575 is about 2mm to 3.5mm. The cushion 50 and the shell 30 are molded as an integral by a process of over-molding, thereby forming a smooth, non-removable seamless connection, so that the product becomes more aesthetic and more reliable. Moreover, the shell 30 can be used in masks 100 in different sizes, and different designs of the masks 100 can be formed by adjusting the size and profile of the cushion 50. In the present embodiment, one side of the nasal contact area 55 away from the shell 30 and one side of the oral contact area 57 away from the shell 30 jointly form a flexible pad surrounding the second opening 53.

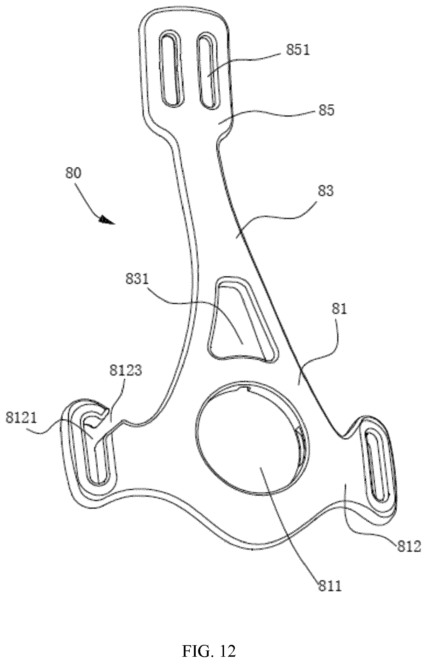

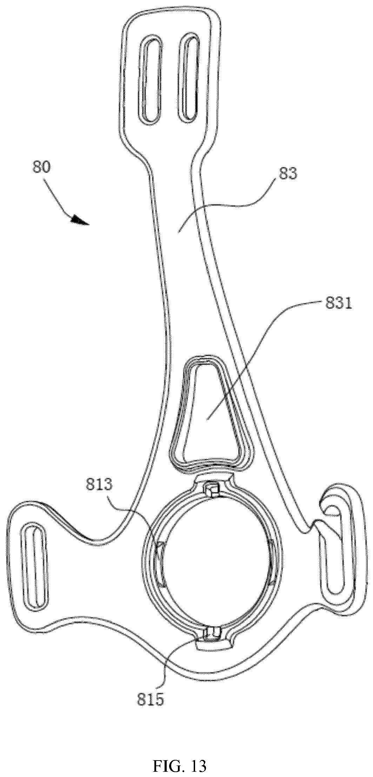

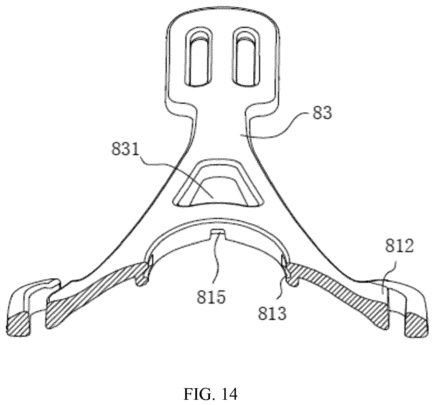

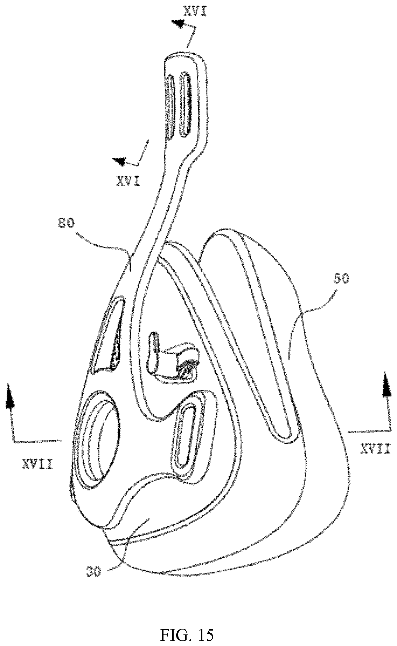

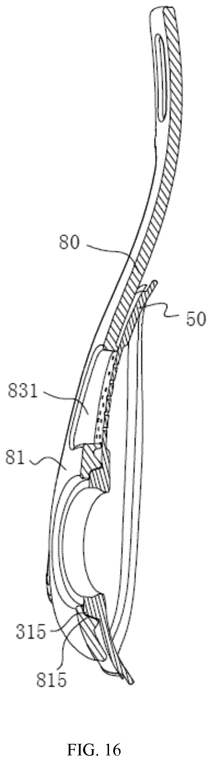

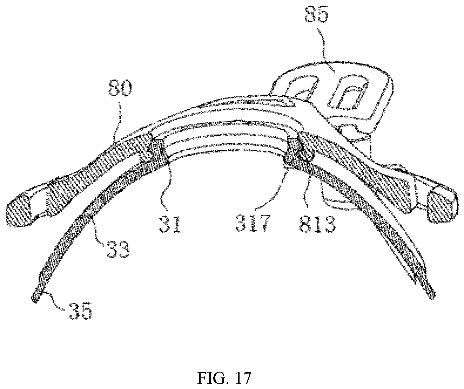

Referring to FIGs. 12 to 14, the frame 80 is disposed on the shell 30, wherein the frame comprises an engaging portion 81, an arc-shaped bridge 83 extending from the engaging portion 81 towards one end, and a connecting end 85 formed at the end of the arc-shaped bridge 83 away from the engaging portion 81. A circular engaging hole 811 is formed in the middle of the engaging portion 81, and extends outwardly from both sides to form pulling portions 812. The engaging hole 811 can be removably sleeved on the shell 30. Two latching holes 813 are formed in the periphery of the engaging hole 811 corresponding to the two pulling portions 812 at a side of the engaging portion 81 towards the shell 30, and two slots 815 are formed concavely corresponding to the arc-shaped bridge 83. The connection line between two latching holes 813 is perpendicular to the connection line between two slots 815. Also referring to FIGs. 15 to 17, two clamping positions 315 and two button positions 317 of the shell 30 are respectively inserted into the two slots 815 and the two latching holes 813. Two pulling portions 812 are suspendingly placed on the face of a person, wherein connection holes 8121 are provided. The connection holes 8121 are substantially square with a length in the range of 18mm to 20mm and a width of about 4mm. One pulling portion 812 of the frame 80 forms an opening 8123 in communication with the connection holes 8121. The opening 8123 is formed at a side of the pulling portion 812 adjacent to the arc-shaped bridge 83. The opening 8123 facilitates removal of the headgear 90 from the corresponding connection holes 8121 or insertion into the corresponding connection holes 8121 manually by a patient, without fully or partly releasing the headgear 90, so that the mask 100 can be easily worn or removed and convenience in use by the patient is improved. It can be construed that it is possible that no opening 8123 is provided in the pulling portion 812.

The arc-shaped bridge 83 is integrally presented as an arc-shaped strip, which bends and extends towards a side of the engaging portion 81 and then bends and extends towards an opposite side, so that it is presented as a flattened "S" shape as a whole. The lengthwise direction of the arc-shaped bridge 83 is parallel to a central connection line between the first shell area 331 and the second shell area 333 of the shell portion 33, and is perpendicular to a central connection line between two pulling portions 812. The arc-shaped bridge 83 further defines apertures 831 adjacent to the engaging portion 81, wherein the apertures 831 are disposed correspondingly in the area of the vent holes 3315 of the shell so that the vent holes 3315 are in communication with the outside. The connecting end 85 is suspendingly placed on the forehead of a person, so that it may drive the arc-shaped bridge 83 when pulled by the headgear 90 to flexibly bend towards one side of the shell 30. The connecting end 85 defines two connection holes 851 parallelly. The connection holes 851 of the connecting end 85 are substantially strip holes with a width of about 3mm to 4mm and a length of about 18mm to 20mm. In this embodiment, the connecting end 85 of the frame 80 is not in contact with the forehead of a person, so the frame 80 is a non-contacting frame, i.e., a three-point suspension frame.

Referring to FIG. 18, the headgear 90 comprises a main portion 91 surrounding the rear side of the head and two first connection straps 93 and two second connection straps 95 formed on the main portion 91. The two first connection straps 93 and the two second connection straps 95 are spaced apart from one another. A fastening portion 931 is formed in each first connection strap 93 adjacent to the end part, and a hook tab 933 is provided at the end part. The hook tab 933 goes through the connection hole 851 of the frame 80 and returns and is fastened to the fastening portion 931. In this embodiment, the fastening portion 931 is a UBL fabric formed on the outer surface of the first connection strap 93. The structure of the two second connection straps 95 is similar to that of the two first connection straps 93, and the two second connection straps 95 are respectively disposed through and connected in two connection holes 8121 of the frame 80. It can be construed that the headgear 90 can be separated from the frame 80 in the event of not wearing the mask.

When the mask 100 is to be assembled, the anti-asphyxia valve 15 is latched to the connector 10, and the elbow 20 is latched to the anti-asphyxia valve 15. The shell 30 is removably latched to the elbow 30, thereby also connecting the cushion 50 to the elbow 30. The frame 80 is latched to the shell 30. The headgear 90 is connected to the frame 80.

When the mask 100 is in use, the main portion 91 of the headgear 90 surrounds the rear side of the wearer's head, the hook tabs 933 of two first connection straps 93 go through the corresponding connection holes 851 in the frame 80 and are fastened to the fastening portion 931, and two second connection straps 95 are connected to two connection holes 8121 of the frame 80, thereby securing the mask 100 in the wearer's oral and nasal contact areas and adjusting the second opening 53 of the cushion 50 so that it corresponds to the nasal contact area of the wearer. The connector 10 is connected to the air delivery pipe. When a positive pressure breathable air enters the elbow 20 through the connector 10, the sealing portion 1531 of the anti-asphyxia valve 15, by the action of a pressure and a flow of a positive pressure air, can be flexibly bent with respect to the handle portion 153 and adhered to the joint face 23 of the elbow, thereby sealing the anti-asphyxia holes 231 and preventing the air flow from escaping from the elbow 20 via the anti-asphyxia holes 231.

As the arc of the frame 80 is specially designed, the connecting end 85 of the frame 80 during wearing would not contact the forehead part of the patient, i.e., the traditional forehead-supporting structure is not used to avoid discomfort caused by long-time wearing. Furthermore, only by adjusting the force to wear two first connection straps 93 and two second connection straps 95 of the headgear 90 can adjust the distance between the connecting end 85 of the whole frame 80 and the forehead of the wearer, thereby adjusting the angle and the level of tightness to wear the mask 100.

In order to satisfy the use requirements of the patients in different age groups, it is often required to design three kinds of products, i.e., large-sized, medium-sized and small-sized products for the patients to choose. In the present case, since the shell 30 of the masks 100 in different sizes are commonly used, the masks 100 in a large size, in a medium size and in a small size are formed by simply adjusting the size and profile of the cushions 50. Therefore, the masks 100 in the large size, in the medium size and in the small size can all share the injection mold of the shell 30 and thus save the cost of molding. Moreover, as the three kinds of masks 100 share the same shell 30, the large-sized, the medium-sized and the small-sized masks 100 can also share the frame 80 that match the shell 30, and thus save the costs of design and manufacture of the frame 80.

The buffer structure 58 of the cushion 50 may adjust the distance between the first buffer member 581 and the second buffer member 583 according to the nasal profile of the wearer, to match the nasal profiles of different users, so that the mask 100 may improve comfort level and sealing performance during wearing under the condition that the matching is satisfied. In addition, when pulled by the headgear 90, the arc-shaped bridge 83 at both sides of the frame 80 can be bent flexibly towards one side of the shell 30 to cushion and balance the pulling force of the headgear 90.

The structural stiffener 551, the opening stiffener 553, the first stiffener 571 and the second stiffener 575 at the inner side of the cushion 50 enhance the structural stability of the cushion 50, and prevent the cushion 10 from collapsing downwards when bearing a force in contact with the nasal skin of the patient, thus leading to problems such as unstable wearing and leakage. Therefore, when the comfort level of skin contact is guaranteed, the stability of the cushion 50 of the single-layered silicone structure can be ensured.

It can be construed that when the frame 80 can be firmly latched to the shell 30, the number and position of the clamping positions 315 and the button positions 317 on the shell 30 can be designed according to the requirements. Meanwhile, the number and position of the slots 815 and two latching holes 813 of the frame 80 need to be correspondingly designed.

It can be construed that when there are no anti-asphyxia holes 231 on the elbow 20, the anti-asphyxia valve 15 can be deleted and the elbow 20 is directly connected to the connector 10.

Although the present invention has been described with reference to the embodiments thereof and the best modes for carrying out the present invention, it is apparent to those skilled in the art that a variety of modifications and changes may be made without departing from the scope of the present invention, which is intended to be defined by the appended claims.

## Claims

1. A mask, comprising:
a connector (10);
an anti-asphyxia valve (15) connected to the connector (10);
an elbow (20) connected to the anti-asphyxia valve (15);
a shell (30) connected to the elbow (20);
a cushion (50) disposed at an end of the shell (30) away from the elbow (20), wherein the cushion (50) defines a first opening (51) and a second opening (53), the first opening (51) faces towards the shell (30), the second opening (53) is away from the shell (30), and the cushion (50) forms a flexible pad (532) on an edge region thereof adjacent to the second opening (53); the cushion (50) comprises a nasal contact area (55) and an oral contact area (57), wherein the nasal contact area (55) concavely forms a buffer structure (58), the buffer structure (58) is an elongated structure extending from a top end of the nasal contact area (55) towards the oral contact area (57) along both sides, the buffer structure (58) comprises a first buffer member (581), a second buffer member (583), and an inwardly concave surface (585) connected to the first buffer member (581) and the second buffer member (583), the first buffer member (581) is adjacent to the first opening (51), the second buffer member (583) is adjacent to the flexible pad (532), and the inwardly concave surface (585) can deform to adjust a distance between the first buffer member (581) and the second buffer member (583); the cushion (50) is made of a silicone rubber, a thickness of the cushion (50) gradually decreases from the first opening (51) to the second opening (53), the thickness has a minimum value of 0.4 mm, the cushion (50) surrounds a periphery of the first opening (51) and concavely forms an embedded slot (511), the embedded slot (511) is closely sleeved on a peripheral wall of the shell (30);
a frame (80) latched to the shell (30), wherein the frame (30) comprises an engaging portion (81), an arc-shaped bridge (83) extending from an end of the engaging portion (81), and a connecting end (85) formed at the arc-shaped bridge (83) away from the end of the engaging portion (81), the engaging portion (81) defines an engaging hole (811) and forms two pulling portions (812) extending outwardly from both sides of the engaging hole (811), respectively, wherein the engaging hole (811) is detachably sleeved on the shell (30); and
a headgear (90) connected to the connecting end (85) and two pulling portions (812) of the frame (80) to assist in wearing of the mask,
wherein when the mask is worn, two pulling portions (812) of the frame (80) are suspendingly placed on a face portion of a wearer and the connecting end (85) is suspendingly placed on a forehead portion, so that the frame (80) is not in contact with the skin of a face or a forehead.

2. The mask of claim 1, **characterized in that** the cushion (50) and the shell (30) are seamlessly and undetachably connected.

3. The mask of claim 1, **characterized in that** the first buffer member (581), the second buffer member (583), and the inwardly concave surface (585) are symmetrically disposed with respect to an axis of the cushion (50), and an inwardly concave depth of the inwardly concave surface (585) gradually decreases along a direction extending from a top end of the nasal contact area (55) to the oral contact area (57).

4. The mask of claim 1, **characterized in that** a plane (A) where a profile line of the inwardly concave surface (585) resides in a lengthwise direction tilts with respect to a central connection line between the first opening (51) and the second opening (53), and the plane (A) is gradually away from the second opening (53) along a direction from the oral contact area (57) to the nasal contact area (55), and a top end of the second buffer member (583) protrudes outwardly with respect to the first buffer member (581).

5. The mask of claim 1, **characterized in that** a curvature radius of a cross-sectional profile of the inwardly concave surface (585) is in a range of 4mm to 40mm, and a maximum distance between the first buffer member (581) and the second buffer member (583) is between 10mm and 16mm.

6. The mask of claim 1, **characterized in that** a thickness of the inwardly concave surface (585) is uniform and is in a range of 0.4mm to 1.5mm.

7. The mask of claim 1, **characterized in that** the nasal contact area (55) is provided with two thickening structural stiffeners (551) and two opening stiffeners (553) adjacent to the buffer structure (58), the two structural stiffeners (553) are symmetrically disposed inside the nasal contact area (55) and are located at a side of the second buffer member (583) away from the first buffer member (581), the two opening stiffeners (553) are symmetrically disposed with respect to each other, and the corresponding structural stiffeners (551) are adjacent to the second opening (53).

8. The mask of claim 7, **characterized in that** the oral contact area (57) defines two thickening first stiffeners (571) inside adjacent to the nasal contact area (55) and a second stiffener (575) away from the nasal contact area (55), the two first stiffeners (571) are symmetrically disposed and respectively adjacent to two structural stiffeners (551) in the nasal contact area (55), and the second stiffener (575) is located between the two first stiffeners (571).

9. The mask of claim 8, **characterized in that** a thickness of the structural stiffener (551) is between 2.5mm and 3mm, a thickness of the opening stiffener (553) is 1mm, a thickness of the first stiffener (571) is between 2.5mm and 4mm, and a thickness of the second stiffener (575) is between 2mm and 3.5mm.

10. The mask of claim 1, **characterized in that** an end of the connector (10) surrounds an inner periphery thereof to form an annular portion (11) by protruding inwardly, the anti-asphyxia valve (15) comprises a valve body (151) and a valve membrane (153) connected to the valve body (151), one end of the valve body (151) surrounds a periphery thereof and protrudes outwardly to form a snap ring (1511), and the other end of the valve body (151) defines two latching holes (1513) at an outer side of the periphery and forms concavely receiving holes (1517) at an inner side of an end face, the snap ring (1511) is inserted into the connector (10) and latched to the annular portion (11) of the connector (10), the valve membrane (153) is made of a silicone rubber material and is partly latched in the receiving holes (1517), an end of the elbow (20) protrudes outwardly from a periphery thereof to form two fasteners (21), and the two fasteners (21) are inserted into the valve body (151) and are latched in two latching holes (1513) of the valve body (151).

11. The mask of claim 10, **characterized in that** the valve membrane (153) comprises a sealing portion (1531) and a handle portion (1533) formed on a periphery of the sealing portion (1531), wherein the sealing portion (1531) is formed as a block at an end of the valve body (151), the handle portion (1533) is inserted and secured in receiving holes (1517) of the valve body (151), an inner wall of the elbow (20) adjacent to an end of the anti-asphyxia valve (15) defines a joint face (23) which tilts with respect to an axis of the elbow (20), the joint face (23) defines two anti-asphyxia holes (231), the anti-asphyxia holes (231) go through a side wall of the elbow (20), the sealing portion (1531) of the anti-asphyxia valve (15) can be flexibly bent with respect to the handle portion (1533) and adhered to the joint face (23) of the elbow (20), thereby sealing the anti-asphyxia holes (231).

## Patentansprüche

1. Maske, umfassend:
ein Verbindungsstück (10);
ein Anti-Asphyxie-Ventil (15), das mit dem Verbindungsstück (10) verbunden ist;
ein Winkelstück (20), das mit dem Anti-Asphyxie-Ventil (15) verbunden ist;
eine Schale (30), die mit dem Winkelstück (20) verbunden ist;
ein Polster (50), das an einem Ende der Schale (30) angeordnet ist, das vom Winkelstück (20) abgewandt ist, worin das Polster (50) eine erste Öffnung (51) und eine zweite Öffnung (53) definiert, die erste Öffnung (51) der Schale (30) zugewandt ist, die zweite Öffnung (53) von der Schale (30) abgewandt ist, und das Polster (50) ein flexibles Kissen (532) an einem Randbereich davon bildet, der an die zweite Öffnung (53) angrenzend ist; das Polster (50) einen Nasenkontaktbereich (55) und einen Mundkontaktbereich (57) umfasst, worin der Nasenkontaktbereich (55) eine Pufferstruktur (58) konkav bildet, die Pufferstruktur (58) eine längliche Struktur ist, die sich vom einem oberen Ende des Nasenkontaktbereichs (55) zum Mundkontaktbereich (57) hin entlang beider Seiten erstreckt, die Pufferstruktur (58) ein erstes Pufferelement (581), ein zweites Pufferelement (583) und eine nach innen konkaven Fläche (585) umfasst, die mit dem ersten Pufferelement (581) und dem zweiten Pufferelement (583) verbunden ist, wobei das erste Pufferelement (581) an die erste Öffnung (51) angrenzend ist, das zweite Pufferelement (583) an das flexible Kissen (532) angrenzend ist, und die nach innen konkave Fläche (585) sich verformen kann, um einen Abstand zwischen dem ersten Pufferelement (581) und dem zweiten Pufferelement (583) anzupassen; das Polster (50) aus einem Silikonkautschuk besteht, eine Dicke des Polsters (50) allmählich von der ersten Öffnung (51) zur zweiten Öffnung (53) sinkt, die Dicke einen Mindestwert von 0,4 mm hat, das Polster (50) einen Umfang der ersten Öffnung (51) umgibt und einen eingebetteten Schlitz (511) konkav bildet, der eingebettete Schlitz (511) auf eine Umfangswand der Schale (30) eng aufgeschoben ist;
einen Rahmen (80), der an der Schale (30) eingerastet ist, worin der Rahmen (30) einen Engriffabschnitt (81), eine bogenförmige Brücke (83), die sich von einem Ende des Engriffabschnitts (81) erstreckt, und ein Verbindungsende (85), das an der bogenförmigen Brücke (83), vom Ende des Engriffabschnitts (81) abgewandt, ausgebildet ist, umfasst, wobei der Engriffabschnitt (81) ein Eingriffsloch (811) definiert und zwei Ziehabschnitte (812) bildet, die sich jeweils nach außen von beiden Seiten des Eingriffslochs (811) erstrecken, worin das Eingriffsloch (811) lösbar auf der Schale (30) aufgeschoben ist; und
einen Kopfgurt (90), der mit dem Verbindungsende (85) und mit zwei Ziehabschnitten (812) des Rahmens (80) zur Hilfe beim Anziehen der Maske verbunden ist,
worin, wenn die Maske angezogen ist, zwei Ziehabschnitte (812) des Rahmens (80) auf einem Gesichtsabschnitt eines Trägers aufhängend platziert sind und das Verbindungsende (85) auf einem Stirnabschnitt aufhängend platziert ist, so dass der Rahmen (80) die Haut eines Gesichts oder eine Stirn nicht berührt.

2. Maske nach Anspruch 1, **dadurch gekennzeichnet, dass** das Polster (50) und die Schale (30) nahtlos und unlösbar verbunden sind.

3. Maske nach Anspruch 1, **dadurch gekennzeichnet, dass** das erste Pufferelement (581), das zweite Pufferelement (583) und die nach innen konkave Fläche (585) im Hinblick auf eine Achse des Polsters (50) symmetrisch angeordnet sind, und eine nach innen konkave Tiefe der nach innen konkaven Fläche (585) entlang einer Richtung allmählich abnimmt, die sich von einem oberen Ende des Nasenkontaktbereichs (55) zum Mundkontaktbereich (57) erstreckt.

4. Maske nach Anspruch 1, **dadurch gekennzeichnet, dass** eine Ebene (A), wo eine Profillinie der nach innen konkaven Fläche (585) in einer Längsrichtung liegt, sich im Hinblick auf eine zentrale Verbindungslinie zwischen der ersten Öffnung (51) und der zweiten Öffnung (53) neigt, und die Ebene (A) sich von der zweiten Öffnung (53) entlang einer Richtung vom Mundkontaktbereich (57) zum Nasenkontaktbereich (55) allmählich entfernt, und ein oberes Ende des zweiten Pufferelementes (583) im Hinblick auf das erste Pufferelement (581) nach außen vorsteht.

5. Maske nach Anspruch 1, **dadurch gekennzeichnet, dass** ein Krümmungsradius eines Querschnittsprofils der nach innen konkaven Fläche (585) in einem Bereich von 4 mm bis 40 mm liegt, und ein Höchstabstand zwischen dem ersten Pufferelement (581) und dem zweiten Pufferelement (583) zwischen 10 mm und 16 mm liegt.

6. Maske nach Anspruch 1, **dadurch gekennzeichnet, dass** eine Dicke der nach innen konkaven Fläche (585) gleichmäßig ist und in einem Bereich von 0,4 mm bis 1,5 mm liegt.

7. Maske nach Anspruch 1, **dadurch gekennzeichnet, dass** der Nasenkontaktbereich (55) mit zwei verdickenden Strukturversteifungen (551) und mit zwei Öffnungsversteifungen (553), die der Pufferstruktur (58) benachbart sind, versehen ist, die zwei Strukturversteifungen (553) innerhalb des Nasenkontaktbereichs (55) symmetrisch angeordnet sind und sich an einer Seite des zweiten Pufferelementes (583) befinden, die vom ersten Pufferelement (581) abgewandt ist, die zwei Öffnungsversteifungen (553) zueinander symmetrisch angeordnet sind, und die entsprechenden Strukturversteifungen (551) der zweiten Öffnung (53) benachbart sind.

8. Maske nach Anspruch 7, **dadurch gekennzeichnet, dass** der Mundkontaktbereich (57) zwei verdickende erste Versteifungen (571) innen an den Nasenkontaktbereich (55) angrenzend und eine zweite Versteifung (575) vom Nasenkontaktbereich (55) abgewandt definiert, die zwei ersten Versteifungen (571) symmetrisch angeordnet sind und jeweils zwei Strukturversteifungen (551) im Nasenkontaktbereich (55) benachbart sind, und die zweite Versteifung (575) zwischen den zwei ersten Versteifungen (571) angeordnet ist.

9. Maske nach Anspruch 8, **dadurch gekennzeichnet, dass** eine Dicke der Strukturversteifung (551) zwischen 2,5 mm und 3 mm beträgt, eine Dicke der Öffnungsversteifung (553) 1 mm beträgt, eine Dicke der ersten Versteifung (571) zwischen 2,5 mm und 4 mm beträgt, und eine Dicke der zweiten Versteifung (575) zwischen 2 mm und 3,5 mm beträgt.

10. Maske nach Anspruch 1, **dadurch gekennzeichnet, dass** ein Ende des Verbindungsstücks (10) einen Innenumfang davon umgibt, um einen ringförmigen Abschnitt (11) zu bilden, indem es nach innen vorsteht, das Anti-Asphyxie-Ventil (15) einen Ventilkörper (151) und eine Ventilmembran (153) umfasst, die mit dem Ventilkörper (151) verbunden ist, ein Ende des Ventilkörpers (151) einen Umfang davon umgibt und nach außen vorsteht, um einen Schnappring (1511) zu bilden, und das andere Ende des Ventilkörpers (151) zwei Rastlöcher (1513) an einer Außenseite des Umfangs definiert und konkav aufnehmende Löcher (1517) an einer Innenseite einer Endfläche bildet, der Schnappring (1511) in das Verbindungsstück (10) eingefügt ist und an dem ringförmigen Abschnitt (11) des Verbindungsstücks (10) eingerastet ist, die Ventilmembran (153) aus einem Silikonkautschukmaterial besteht und teilweise in den aufnehmenden Löchern (1517) eingerastet ist, ein Ende des Winkelstücks (20) nach außen aus einem Umfang davon vorsteht, um zwei Befestigungselemente (21) zu bilden, und die zwei Befestigungselemente (21) in den Ventilkörper (151) eingefügt sind und in zwei Rastlöchern (1513) des Ventilkörpers (151) eingerastet sind.

11. Maske nach Anspruch 10, **dadurch gekennzeichnet, dass** die Ventilmembran (153) einen Dichtungsabschnitt (1531) und einen Griffabschnitt (1533) umfasst, der an einem Umfang des Dichtungsabschnitts (1531) geformt ist, worin der Dichtungsabschnitt (1531) als ein Block an einem Ende des Ventilkörpers (151) ausgebildet ist, der Griffabschnitt (1533) in Aufnahmelöchern (1517) des Ventilkörpers (151) eingefügt und gesichert ist, eine Innenwand des Winkelstücks (20), die an ein Ende des Anti-Asphyxie-Ventils (15) angrenzend ist, eine Fügefläche (23) definiert, die sich im Hinblick auf eine Achse des Winkelstücks (20) neigt, die Fügefläche (23) zwei Anti-Asphyxie-Löcher (231) definiert, die Anti-Asphyxie-Löcher (231) eine Seitenwand des Winkelstücks (20) durchlaufen, der Dichtungsabschnitt (1531) des Anti-Asphyxie-Ventils (15) flexibel im Hinblick auf den Griffabschnitt (1533) gebogen werden kann und an der Fügefläche (23) des Winkelstücks (20) anhaften lassen werden kann, wodurch die Anti-Asphyxie-Löcher (231) abgedichtet werden.

## Revendications

1. Masque, comprenant :
un connecteur (10) ;
une valve anti-asphyxie (15) connectée au connecteur (10) ;
un coude (20) connecté à la valve anti-asphyxie (15) ;
une coque (30) connectée au coude (20) ;
un coussinet (50) disposé à une extrémité de la coque (30) éloignée du coude (20), dans lequel le coussinet (50) définit une première ouverture (51) et une seconde ouverture (53), la première ouverture (51) est orientée vers la coque (30), la seconde ouverture (53) est éloignée de la coque (30), et le coussinet (50) forme un tampon flexible (532) sur une région de bord de celui-ci adjacente à la seconde ouverture (53) ; le coussinet (50) comprend une zone de contact nasal (55) et une zone de contact buccal (57), dans lequel la zone de contact nasal (55) forme de manière concave une structure de tampon (58), la structure de tampon (58) est une structure allongée s'étendant depuis une extrémité supérieure de la zone de contact nasal (55) vers la zone de contact buccal (57) le long des deux côtés, la structure de tampon (58) comprend un premier élément de tampon (581), un second élément de tampon (583) et une surface concave vers l'intérieur (585) connectée au premier élément de tampon (581) et au second élément de tampon (583), le premier élément de tampon (581) est adjacent à la première ouverture (51), le second élément de tampon (583) est adjacent au coussinet flexible (532) et la surface concave vers l'intérieur (585) peut se déformer pour ajuster une distance entre le premier élément de tampon (581) et le second élément de tampon (583) ; le coussinet (50) est en caoutchouc de silicone, une épaisseur du coussinet (50) diminue progressivement de la première ouverture (51) vers la seconde ouverture (53), l'épaisseur a une valeur minimum de 0,4 mm, le coussinet (50) entoure une périphérie de la première ouverture (51) et forme de manière concave une fente encastrée (511), la fente encastrée (511) est manchonnée de manière étroite sur une paroi périphérique de la coque (30) ;
un cadre (80) verrouillé à la coque (30), dans lequel le cadre (30) comprend une partie de mis en prise (81), un pont en forme d'arc (83) s'étendant depuis une extrémité de la partie de mise en prise (81), et une extrémité de connexion (85) formée sur le pont en forme d'arc (83) loin de l'extrémité de la partie de mise en prise (81), la partie de mise en prise (81) définit un trou de mise en prise (811) et forme deux parties de traction (812) s'étendant vers l'extérieur depuis les deux côtés du trou de mise en prise (811), respectivement, dans lequel le trou de mise en prise (811) est manchonné de manière amovible sur la coque (30) ; et
une garniture de tête (90) connectée à l'extrémité de connexion (85) et deux parties de traction (812) du cadre (80) pour aider au port du masque,
dans lequel lorsque le masque est porté, deux parties de traction (812) du cadre (80) sont placées de manière suspendue sur une partie faciale d'un utilisateur et l'extrémité de connexion (85) est placée suspendue sur une partie frontale, de sorte que le cadre (80) n'est pas en contact avec la peau d'un visage ou d'un front.

2. Masque selon la revendication 1, **caractérisé en ce que** le coussinet (50) et la coque (30) sont connectés sans couture et de manière inamovible.

3. Masque selon la revendication 1, **caractérisé en ce que** le premier élément de tampon (581), le second élément tampon (583) et la surface concave vers l'intérieur (585) sont disposés symétriquement par rapport à un axe du coussinet (50), et une profondeur concave vers l'intérieur de la surface concave vers l'intérieur (585) diminue progressivement le long d'une direction s'étendant depuis une extrémité supérieure de la zone de contact nasal (55) vers la zone de contact buccal (57).

4. Masque selon la revendication 1, **caractérisé en ce qu'**un plan (A) où une ligne de profil de la surface concave vers l'intérieur (585) réside dans une direction longitudinale s'incline par rapport à une ligne de connexion centrale entre la première ouverture (51) et la seconde ouverture (53), et le plan (A) s'éloigne progressivement de la seconde ouverture (53) le long d'une direction allant de la zone de contact buccal (57) à la zone de contact nasal (55), et une extrémité supérieure du second élément de tampon (583) fait saillie vers l'extérieur par rapport au premier élément de tampon (581).

5. Masque selon la revendication 1, **caractérisé en ce qu'**un rayon de courbure d'un profil de section transversale de la surface concave vers l'intérieur (585) est dans une plage de 4 mm à 40 mm, et une distance maximum entre le premier élément de tampon (581) et le second élément de tampon (583) est comprise entre 10 mm et 16 mm.

6. Masque selon la revendication 1, **caractérisé en ce qu'**une épaisseur de la surface concave vers l'intérieur (585) est uniforme et se situe dans une plage de 0,4 mm à 1,5 mm.

7. Masque selon la revendication 1, **caractérisé en ce que** la zone de contact nasal (55) est pourvue de deux raidisseurs structurels épaississants (551) et de deux raidisseurs d'ouverture (553) adjacents à la structure de tampon (58), les deux raidisseurs structurels (553) sont disposés symétriquement à l'intérieur de la zone de contact nasal (55) et sont positionnés sur un côté du second élément de tampon (583) éloigné du premier élément de tampon (581), les deux raidisseurs d'ouverture (553) sont disposés symétriquement l'un par rapport à l'autre, et les raidisseurs structurels correspondants (551) sont adjacents à la seconde ouverture (53).

8. Masque selon la revendication 7, **caractérisé en ce que** la zone de contact buccal (57) définit deux premiers raidisseurs épaississants (571) adjacents intérieurement à la zone de contact nasal (55) et un second raidisseur (575) éloigné de la zone de contact nasal (55), les deux premiers raidisseurs (571) sont disposés symétriquement et respectivement adjacents à deux raidisseurs structurels (551) dans la zone de contact nasal (55), et le second raidisseur (575) est situé entre les deux premiers raidisseurs (571).

9. Masque selon la revendication 8, **caractérisé en ce qu'**une épaisseur du raidisseur structurel (551) est comprise entre 2,5 mm et 3 mm, une épaisseur du raidisseur d'ouverture (553) est de 1 mm, une épaisseur du premier raidisseur (571) est comprise entre 2,5 mm et 4 mm, et une épaisseur du second raidisseur (575) est comprise entre 2 mm et 3,5 mm.

10. Masque selon la revendication 1, **caractérisé en ce qu'**une extrémité du connecteur (10) entoure une périphérie intérieure de celui-ci pour former une partie annulaire (11) en faisant saillie vers l'intérieur, la valve anti-asphyxie (15) comprend un corps de valve (151) et une membrane de valve (153) connectée au corps de valve (151), une première extrémité du corps de valve (151) entoure une périphérie de celui-ci et fait saillie vers l'extérieur pour former un anneau élastique (1511), et l'autre extrémité du corps de soupape (151) définit deux trous de verrouillage (1513) sur un côté extérieur de la périphérie et forme des trous de réception concaves (1517) sur un côté intérieur d'une face d'extrémité, l'anneau d'encliquetage (1511) est inséré dans le connecteur (10) et verrouillé sur le partie annulaire (11) du connecteur (10), la membrane de valve (153) est réalisée en un matériau en caoutchouc de silicone et est partiellement verrouillée dans les trous de réception (1517), une extrémité du coude (20) fait saillie vers l'extérieur à partir d'une périphérie de celui-ci pour former deux fixations (21), et les deux fixations (21) sont insérées dans le corps de valve (151) et sont verrouillées dans deux trous de verrouillage (1513) du corps de valve (151).

11. Masque selon la revendication 10, **caractérisé en ce que** la membrane de valve (153) comprend une partie d'étanchéité (1531) et une partie de poignée (1533) formée sur une périphérie de la partie d'étanchéité (1531), dans lequel la partie d'étanchéité (1531) est formée sous la forme d'un bloc à une extrémité du corps de valve (151), la partie de poignée (1533) est insérée et fixée dans des trous de réception (1517) du corps de valve (151), une paroi intérieure du coude (20) adjacente à un extrémité de la valve anti-asphyxie (15) définit une face de jonction (23) qui s'incline par rapport à un axe du coude (20), la face de jonction (23) définit deux trous anti-asphyxie (231), les trous anti-asphyxie (231) passent à travers une paroi latérale du coude (20), la partie d'étanchéité (1531) de la valve anti-asphyxie (15) peut être pliée de manière flexible par rapport à la partie de poignée (1533) et mise en adhérence à la face de jonction (23) du coude (20), en scellant ainsi les trous anti-asphyxie (231).
